# EUROPÄISCHE PATENTSCHRIFT

(11) **EP 3 231 453 B1**
(45) Veröffentlichungstag und Bekanntmachung des Hinweises auf die Patenterteilung: **25.12.2019**
(21) Anmeldenummer: 17166712.4
(22) Anmeldetag: 14.04.2017
(51) Int. Cl.: A61L 27/02, A61L 24/02

(54) **BONE-SIALOPREOTEIN(BSP)-FUNKTIONALISIERTE KNOCHENERSATZKÖRPER**
BONE SIALOPROTEIN(BSP)-FUNCTIONALISED BONE REPLACEMENT BODIES
BONE SIALO PROTEINE (BSP) FONCTIONNEL REMBLACEMENTS DES OS

(30) Priorität: 14.04.2016 DE 102016106961; 04.08.2016 DE 102016114497
(43) Veröffentlichungstag der Anmeldung: 18.10.2017
(73) Patentinhaber: Immundiagnostik AG, 64625 Bensheim (DE)
(72) Erfinder: BARANOWSKI, Andreas, 55131 Mainz (DE); ROMMENS, Pol Maria, 55131 Mainz (DE); RITZ, Ulrike, 55131 Mainz (DE); KLEIN, Anja, 55131 Mainz (DE); HOFMANN, Alexander, 55131 Mainz (DE); ARMBRUSTER, Franz Paul, 61625 Bensheim (DE)
(74) Vertreter: Benedum, Ulrich Max

(56) Entgegenhaltungen:
- WO-A1-2015/059240
- US-B1- 6 458 763

## Beschreibung

### GEBIET DER ERFINDUNG

Die Erfindung betrifft funktionalisierte Oberflächen und Biomaterialien in der rekonstruktiven Chirurgie des Bewegungsapparates und in der Dentalmedizin.

### HINTERGRUND DER ERFINDUNG

US 5 478 237 (Ishikawa) offenbart ein Knochenimplantat, das mit Hydroxylapatit (HA) beschichtet ist, WO 02/078759 (Stratec Medical AG) ein Knochenersatzmaterial aus einem Gemisch mit amorphem und nanokristallinem Calciumphosphat sowie Hydroxylapatit, WO 02/085250 (Keramed GmbH) ein Material mit resorbierbaren Calciumphosphatphasen, fakultativ, mit adsorbierten Adhäsions- und Signalproteinen wie Bone Morphogenic Protein (BMP), Bone-Sialoprotein (BSP II), Fibronektin, Osteopontin (OPN), ICAM-I, VCAM und deren Derivaten. EP 1 166 804 A2 (Merck Damstadt), WO 99/08730 Medical Center Corporation), DE 100 37 850 A1 (Jenissen H), WO 03/059407 A1 (Straumann Holding AG) beschreiben Knochenersatzmaterialien mit Ubiquitin, Transforming Growth Factor (TGF) oder systemischen Hormonen wie Osteostatin und Osteogenic Growth Peptid (OGP). US 7 229 545 B2 (Biomet Deutschland GmbH) beansprucht eine mit Calciumphosphat mineralisierte Kollagenmatrix. EP 1 442 755 A1 (Depuy Products) Keramiken, beschichtet mit Osteogenic Protein-1 (OP-1), Bone Morphogenic Protein-7 (BMP-7) und anderen nichtkollagenen Proteinen der Knochenmatrix. Osteogene Aktivität wird auch zugeschrieben: Fibroblastenwachstumsfaktor (FGF), Transforming Growth Factor-3 (TGF-p), Plateletderived Growth Factor (PDGF), Insulin Growth Factor (IGF) und deren Familien. Die Bone Morphogenic Proteine (BMP) sind aber die einzigen bekannten Wachstumsfaktoren, welche eine Knochenbildung gesichert unterstützen.

EP1084719 (Depuy Orthopeadics Inc), US 6,45 8,763 (Peterson et al.) WO 2011/000970, WO03/047646 (Inion Ltd.), WO 94/13310 (Höök et al) und WO 2005/104988 (Armbruster et al) offenbaren Gemische mit Tricalciumphosphat, resorbierbarem Polymer und als bioaktive Komponente BSP bzw. unterglykosiliertes BSP.

Die vorgenannten Knochenersatzmaterialien werden zumeist als plastisches oder pastöses Füllmaterial angeboten. Die rekonstruktive Chirurgie benötigt jedoch auch formfeste Knochenersatzkörper für die sofortige Reparatur von Knochendefekten sowie auch formfeste Knochenersatzteile, die nach dem Setzen weiter aushärten und die im Verlauf der Heilung resorbiert werden. Es ist also gesucht, ein formfester Knochenersatzkörper bzw. ein aushärtendes Knochenersatzmaterial, das resorbiert wird sowie die Knochenheilung, den Knochenumbau und das Knochenwachstum, fördern. Der Stand der Technik repräsentiert das Problem.

### KURZE BESCHREIBUNG DER ERFINDUNG

Als Lösung wird hier bereitgestellt ein form- und strukturfester Knochenersatzkörper aus mineralischem Calciumphosphatzement mit verbleibender hydraulischer Aktivität, enthaltend einen Anteil an abgebundenem mineralischen Calciumphosphatzement und einen Anteil an nicht-abgebundenem hydraulisch reaktiven Calciumphosphatzement, dadurch gekennzeichnet, dass der Knochenersatzkörper aus Zementsträngen mit interkonnektierenden Poren aufgebaut ist, dass nach dem Setzen ein hydraulisches Abbinden und Härten der Stränge folgt, und dass auf der hydraulisch abgebundenen, gehärteten Oberfläche und in den Poren des Ersatzkörpers, der im Wesentlichen gegenüber Proteinen inert ist, durch Physissorption humanes Bone-Sialoprotein aufgebracht und gebunden ist. Vor Physisorption des humanen Bone-Sialoprotein können andere neutrale globuläre Proteine adsorbiert werden, die nicht aus dem Komplementsystem stammen. Weitere vorteilhafte Ausführungsformen sind in den Unteransprüchen und in den Beispielen dargestellt.

In einer bevorzugten Ausführungsform besitzt der form- und strukturfeste Knochenersatzkörper eine Gesamtporosität zwischen 20 und 90%. Weiterhin kann das Knochenersatzmaterial des Strukturkörpers zur Verbesserung der Strukturfestigkeit β-Tricalciumphosphat-Granula enthalten. Die einzelnen Granula können einen Durchmesser von mehr als 100 Mikrometer haben. Besonders bevorzugt ist ein strukturfester Knochenersatzkörper in der Gestalt eines gedruckten 3D-Formkörpers. Das hydraulische Knochenersatzmaterial kann die Feststoffe als feine Pulver suspendiert/dispergiert enthalten, wobei mindestens 10% der Feststoffe eine Partikelgröße < 10 µm und mindestens 10% der Feststoffe eine Partikelgröße > 50 µm aufweisen. Auf dem hydraulischen, partiell abgebundenen Knochenersatzmaterial kann durch Physisorption Bone-Sialoprotein gebunden sein, da das Ersatzmaterial chemisch bereits im Wesentlichen inert ist und insbesondere keine Calciumionen mehr abgibt.

Ein weiterer Aspekt betrifft die Verwendung eines derartigen strukurfesten Knochenersatzkörpers bzw. des Knochenersatzmaterials in der rekonstitutiven Chirurgie und in der Dentalmedizin.

Ein Aspekt der Offenbarung betrifft die Verwendung der formstabilen Knochenzementkörpers nach einem der vorstehenden Ansprüche zur Herstellung von alloplastischen Implantaten. Weitere bevorzugte Ausführungsformen der Erfindung ergeben sich aus deren detaillierten Beschreibung, den Abbildungen und den anhängenden Beispielen.

### KURZE BESCHREIBUNG DER ABBILDUNGEN

Es zeigt:
- Fig. 1: eine zeichnerische Darstellung des Versuchs.
- Fig. 2: ein Säulendiagramm zur quantiativen Bindung und Physissorption von BSP auf dem gewählten CPC-KEM und nach einem ersten Waschschritt und nach drei Tagen in einer Pufferlösung;
- Fig. 3A-C: Mikroskopaufnahmen nach BSP Physisorption: (A) - nach Zellaussaat von humanen Osteoblasten (in 4x Vergrößerung); (B) - markiert mit Zelltracker-Rot (in 4x Vergrößerung) und (C) einen Tag nach Zellaussaat (in 2x Vergrößerung), wobei auf der rechten Bildseite der Formkörpers BSP adsorbiert hat und auf der linken Seite nicht;
- Fig. 4A: Mikroskopaufnahmen von Knochenersatzmaterialien, auf denen mit unterschiedlicher Konzentration Fluorescein-markiertes BSP adsorbiert wurde. Von oben nach unten: Physisorption mit 50 µg/mL, 100 µg/mL und 200 µg/ml Fluorescein-markierte BSP-Lösung auf CPC-Knochenersatzmaterial (KEM);
- Fig. 4B: Rasterelektronenmikroskopaufnahmen der verschiedenen KEM-Formkörpern (Plättchen und Zylinder) und eine 100 000 x vergrößerte Darstellung der Oberflächenstuktur;
- Fig. 4C: zeichnerische Darstellung der Schritte zur Herstellung von formfesten Knochenzementkörper mit interkonnektierenden Poren für eine Physisoption von BSP.
- Fig. 4D: Rasterelektronenmikroskopaufnahmen von Knochenzementkörpern aus calcium-defizienten Hydroxlapatit (CDHA); links: Scheiben für *in vitro* Versuche, und rechts: Zylinder für *in vivo* Implantate in der Maus (Vergrößerungen: 10 000x und 50 000 x).
- Fig. 5: Diagramm der Kurve mit den prozentualen Restmenge BSP;
- Fig. 6: Diagramm zur Proliferation humaner Osteoblasten auf Calciumphosphatzement (CPC) ohne (Kontrolle) und mit adsorbiertem BSP nach 3, 5, 7, 10 Tagen nach Zellaussaat - Physisorption mit 50 µg/mL bzw. 200 µg/mL BSP in der Lösung;
- Fig. 7: Fluoreszenzmikroskopaufnahmen humaner Osteoblasten auf CPC-Scheiben. Physisorption von BSP bei ansteigenden Konzentrationen; Färbung der humanen Osteoblasten (hOB) mit Calcein AM nach 14 Tagen Proliferationszeit (Maßstab = 1000 µm);
- Fig. 8: Diagramm mit einer Analyse der Genexpression 14 Tage nach Aussaat der humanen Osteoblasten (hOB) auf CPC mit und ohne physisorbiertem BSP (50 µg/mL und 200 µg/mL BSPg in Phosphatpuffer) - Transkriptionsfaktoren und Marker: osterix (SP7), secreted protein, acidic, cysteine-rich (SPARC), Runt-related transcription factor 2 (Runx2), osteopontin (OPN), alkalische Phosphatase (ALP - Ostase); gestrichelte Linie zeigt den Wert für unbehandelte KEMs (Kontrolle);
- Fig. 9A: zeichnerische Darstellung der Schritte des in-vivo Versuchs (Vergleich mit bone morphogenic protein-7 (BMP-7); CPC = Calciumphosphatzement als KEM;
- Fig. 9B: fotografische Darstellung des Knochenzementkörpers mit interkonnektierenden Poren;
- Fig. 9C: Prinzipdarstellung der Dickenmessung beim runden Gerüstteil-KEM - "Scaffold";
- Fig. 9D: fotografische Darstellung der Implantation des Knochenzementkörpers im Bohrlochdefekt in der Kalotte der Maus;
- Fig. 9E: Fotografie des Kopfes einer enthaarten Maus, deren Bohrlochdefekte mit BSP-CPC-Knochenzementkörpern geschlossen wurden.
- Fig. 10: µ-CT-Aufnahmen post-mortem der Kalotten und der Bohrlochdefekte 8 Wochen nach Operation (Beispiele).
- Fig. 11A-B: Darstellung der Knochendickemessung an der Implantat-Grenzfläche und der Messpunkte für die Knochendicke (Kreise) pro Defekt.
- Fig. 12A-D: Aufnahmen von histologischen Schnitten der Knochenzementkörper nach Hämatoxylin-Eosin-Färbung. Defektheilung: 8 Wochen nach Implantation eines Knochenersatzmaterials mit BMP-7-Belegung im Bohrlochdefekt einer Mauskalotte;
- Fig. 13: ein Diagramm mit einer graphischen Darstellung der Auswertung von Knochenvolumen zu Gesamtvolumen im Maus-Kalotten-Bohrlochdefekt-Modell.

### EINGEHENDE BESCHREIBUNG

Es wird bereitgestellt ein im 3-Druck herstellbarer form- und strukturfester Knochenzementkörper mit interkonnektierenden Poren, auf dem humanes Bone-Sialoprotein (hBSP) als nichtkollagenes bioaktives Knochenmatrixprotein aufgebracht ist. Der Knochenersatzkörper bzw. das Knochenersatzmaterial enthält einen Anteil an mineralischem Knochenzement, der hydraulisch abgebunden hat, und einen Anteil an nicht abgebundenem Knochenzement. Ein solches Knochenersatzmaterial kann im 3-D-Druckverfahren in einen formfesten Formkörper mit interkonnektierenden Poren spritzgegossen bzw. gedruckt werden. Wegen der Abbindereaktion gibt der Körper nach dem hydraulischen Aushärten keine Calciumionen ab und er verhält sich inert, so dass auf dessen Oberflächer bioaktive Proteine adsorbiert werden können, selbst wennn diese sich sofort mit freiem Calcium umsetzen. Es wird offenbart, auf der Oberfläche und in den Poren eines solchen partiell abgebundenen Knochenzementkörpers durch Physisorption humanes BSP aufzubringen, denn es präzipiert dort nicht mit vorhandenem Calcium, sondern es bleibt auf der Oberfläche des partiell abgebundenen Knochenzementkörper und nach Setzen im Knochendefekt bioaktiv. Die Bioaktivität liegt hierbei in der Beteiligung an der Differenzierung von mesenchymalen Stammzellen zu Osteoblasten und Osteoklasten, welche für Knochenneubildung und insbesondere eine Resorption des heterotopen Knochenersatzmaterials erforderlich sind. Dies ist überraschend, aber dadurch erklärlich, dass die hydraulische Abbindereaktion bei einem so hergestellten Knochenzementkörper von Außen in das Innere des Körpers voranschreitet, so dass die Stränge an Oberfläche zuerst aushärten und chemisch gegenüber Proteinen wie BSP inert werden. Dennoch wird der Knochenzement resorbiert, aber diese Resorption erfolgt auf zellulärem Weg.

Es wird weiterhin offenbart, ein Körper aus hydraulisch abgebundenen und partiell nicht-abgebundenen Knochenzement, auf dem Plasma- und Serumproteine adsorbiert sind. Die Adsorption von Plasma- und Serumproteinen auf dem Knochenzementkörper verbessert eine anschließende Physisorption von bioaktivem Bone-Sialoprotein. Die Präadsorption von Proteinen des Komplementsystems wird hingegen als nachteilig angesehen, da diese Protein vielfältige Wirkungen besitzen, regulatorische und inflammatorischen Wirkungen, welche nicht kontrollierbar und vorhersehbar sind. Die Gegenwart von Faktor H aus dem Komplementsystem ist zu vermeiden, da dieser Faktor das BSP bindet. Eine Voradsorption mit aufgereinigten neutralen Plasmaprotein wie Albumin ist bevorzugt. Wahrscheinlich reagieren Plasmaproteine wie Albumin mit reaktiven Oberflächengruppen auf dem Knochenzement und verhindern so, dass das negative aufgeladene Bone-Sialoprotein deaktiviert wird. Das Bone-Sialoprotein kann dann im Gleichgewicht die anderweitig adsorbierten Plasmaproteine auf dem Knochenersatzmaterial verdrängen. Weiterhin ist bevorzugt, den Knochenzementkörper mit einem citrat- oder EDTA-haltigen Puffer vor einer Physisorption von hBSP kurz zu waschen, um anhaftende freie Calcium und Calciumionen abzulösen. Es können natürlich auch andere Chelatreagenzien zum Abwaschen des hydraulischen abgebundenen Knochenzementkörpers verwendet werden.

Das humane Bone-Sialoprotein (hBSP) liegt in Lösung und auf der Oberfläche des Knochenersatzmaterials wegen der starken negativen Ladung im N-terminalen Bereich im Wesentlichen ungefaltet vor. Es besitzt räumliche getrennte Bereiche für die Bindung von Kollagen, die Nukleus-Eigenschaft für die Bildung Hydroxylapatit und für die zellbindende Aktivierung und Differenzierung von Osteoblasten durch das Integrin-bindende Motiv RGD im Bereich des C-terminalen globulären Endes. Die Nukleuswirkung und die Bildung von Hydroxylapatit (HA) ist bedingt durch die Glutamatreichen Domänen in der aminoterminalen Hälfte des BSP; siehe Tye CE et al, in Delineation of the Hydroxyapatite-nucleating Domains of Bone Sialoprotein, JBC 2003, 278:7949-7955. Eine solche Nukleuswirkung und Calciumphosphat präzipitierende Wirkung kann auch mit künstlichem Poly-Glutamat erreicht werden. Es wird daher auch vorgeschlagen, den Knochenzementkörper mit synthetischem Poly(Glu) vorzubehandeln, allein oder zusammen mit Poly(Lys), Poly(Gly-Pro-Hyp), Tropocollagen oder Gelatine.

Der Knochenzementkörper wird formfest mit einer Gesamtporosität zwischen 20 und 90% hergestellt. Eine so hohe Gesamtporosität bei gleichzeitiger Formstabilität ist erreichbar, wenn man den Körper dreidimensional im 3-D-Druck aus netzförmig gelegten Strangbahnen zu einem formfesten Gerüst aufbaut - die interkonnektierenden Poren resultieren zwangsläufig. Die Strangbahnen werden aus partiell abgebundenen Knochenzement gespritzt, und dann der so hergestellte Knochenzementkörper hydraulisch gehärtet. Eine bevorzugte Ausführungsform ist somit ein Knochenzementkörper in Gestalt eines gedruckten 3D-Formkörpers aus partiell abgebundenen hydraulischen Knochenzement, auf dem nach dem Aushärten Protein und insbesondere bioaktives humanes BSP aufgebracht ist.

In einer alternativen Ausführungsform wird ein Knochenersatzmaterial eingesetzt, bestehend aus β-TCP-Granula und resorbierbaren Polymer oder Copolymer, zum Beispiel Methyl-Methacrylat (PMMA) oder Polylactid-co-Glycolid (PLGA). PLGA ist eine organische Substanz auf Milchsäurebasis, die vom menschlichen Körper rasch resorbiert wird. Die einzelnen β-TCP-Granula können einen Durchmesser von mehr als 100 Mikrometer besitzen. Der Knochenzementkörper wird dann aus einem Tricalciumphosphatgranulat-Polymer-Gemisch gespritzt bzw. gegossen.

In der Medizin und Zahnmedizin werden auch Calcium- und Magnesiumsalze der Orthophosphorsäure verwendet. Der sogenannte Phosphatzement ist beispielsweise in der Zahnmedizin eines der am häufigsten gebrauchten Materialien. Der Zement wird aus einem Pulver von Zinkoxid und Magnesiumoxid mit Orthophosphorsäure angerührt und ergibt nach dem Abbinden einen Zement, der als Unterfüllung oder provisorische Füllung eingesetzt wird. Die Phosphatzemente umfassen auch solche, die neben Ca- und Mg-Salzen, -oxide und -hydroxide andere Ionen enthalten, insbesondere Na⁺, K⁺, NH₄⁺ und/oder solche die Zn²⁺ enthalten. Umfasst sind auch die Ca- und Mg-Salze der Glycerophosphorsäure und die anderer mono- oder di-substituierter organischer Phosphorsäureester. Bevorzugt sind Knochenersatzmaterialien, deren suspendierte mineralischen Feststoffe zu größer 50% aus Calcium- und/oder Magnesiumphosphaten bestehen. Daneben können die Feststoffe übliche Füllstoffe, wie z.B. CaCO₃ und/oder SrCO₃ enthalten, oder auch BaCO₃ als Röntgenopaker.

Bevorzugte Magnesiumverbindungen, wasserfrei oder als Hydrat, sind Magnesiumhydrogenphosphat (MgHPO₄), Trimagnesiumphosphat (Mg₃(PO₄)₂), Magnesiumdihydrogenphosphat (Mg(H₂PO₄), Magnesiumchlorid (MgCl₂), Magnesiumglycerophosphat. Magnesiumhydroxid (Mg(OH)₂), Magnesiumhydroxidcarbonat, Magnesiumoxid (MgO), Magnesiumcitrate, Calcium-Magnesiumcarbonat und/oder deren Gemische.

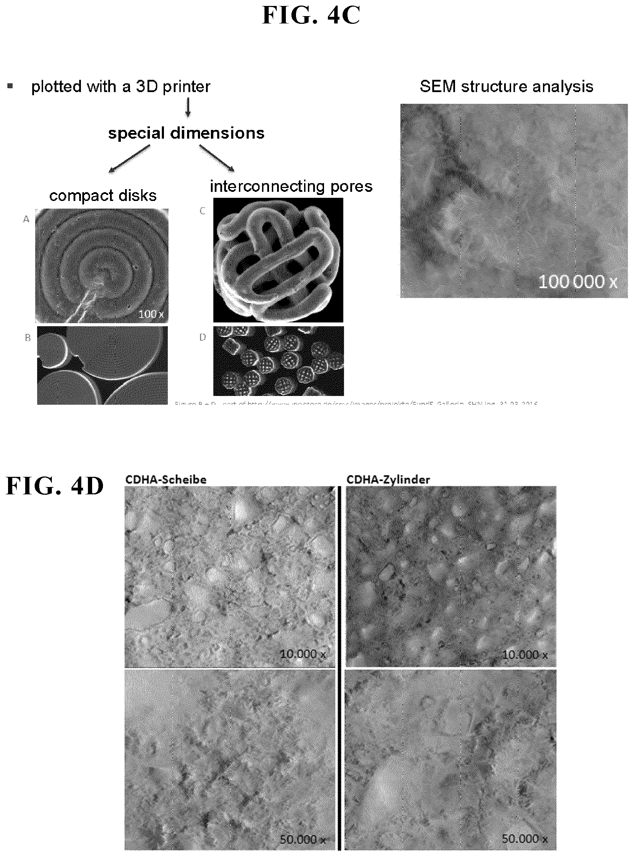

Figur 4C zeigt Knochenzementkörper, wie sie mit einem 3D-Drucker in den Maßen und der Gestalt eines Knochendefekts gedruckt werden können. Das für den 3-D-Druck verwendete Knochenersatzmaterial ist beispielsweise formbarer Calciumphosphatzement (CPC), der erhältlich ist durch Anmischen eines reaktiven mineralischen Knochenzements mit einer Trägerträgerflüssigkeit. Es folgt eine Formgebung in einem 3-D-Druckverfahren und ein Abbinden und Härten des Formkörpers in Gegenwart von pharmakologisch akzeptablen Stoffen. Der Abindeprozesses kann dann durch Entzug des Wassers gestoppt werden. Geeignete Knochenzemente beschreiben Hofmann MP et al in Carvable calcium phosphate bone substitute material, JBMR, Part B: Applied Biomaterials 2007, 83B:1-8; DOI: 10.1002/jbm.b.3076, oder Lode A et al in Fabrication of porous scaffolds by three-dimensional plotting of a pasty calcium phosphate bone cement under mild conditions, JTERM 2012, 8:682-693, DOI: 10.1002/term.1563. Sie lehren auch ein Drucken von Körpern mit interkonnektierenden Poren aus mineralischen Knochenzementen, die bei Kontakt mit wässriger Lösung zu soliden Materialien aushärten. Die hydraulische Aktivität ist aus medizinischer Sicht interessant, weil die Abbindereaktion partiell auch nach der Implantation unter physiologischen Bedingungen weitergehen kann. Die hydraulische Aktivität bezeichnet die Fähigkeit des Materials in Gegenwart von Wasser oder einer wasserhaltigen Komponente chemisch weiterzulaufen, die aer auch durch Wasserentzug vor Erreichen der vollständigen Umsetzung abgebrochen werden kann. Der erneute Kontakt mit Wasser bewirkt eine Hydratation der reaktiven Zementkomponente und der unterbrochene Abbindeprozess läuft weiter. Ein Auslassen eines keramischen Sinterschrittes ist auch beschrieben in der WO 2015/059240 A1 (Innotere GmbH, DE).

Es wird weiterhin ein Aufbringen von BSP auf den CPC-Körper mit interkonnektierenden Poren durch Physisorption beschrieben. Bei der Physisorption sind die Kräfte schwach, da charakteristisch keine chemischen Bindungen eingegangen werden. Die Physisorption von BSP erfolgt auf dem hydraulisch partiell abgebundenen Knochenzementkörper bevorzugt nach vorheriger Physisorption von Albumin, Globulin und kleineren Plasmaproteinen, welche selbst nicht mit BSP reagieren oder binden. Störende Proteine sind Proteasen und Proteine aus dem Komplementsystem wie Faktor H. Die kleinen Plasmaproteine diffundieren in den Knochenzementkörper mit den interkonnektierenden Poren und reagieren ab mit reaktiven Oberflächenstellen, so dass im Nachgang das humane BSP mit pK 4,0 besser adsorbiert wird. Die wichtigste Eigenschaft der Physisorption ist die geringe Veränderung des Adsorbats bzw. des gebundenen Proteins.

Nachteilig an herkömmlichen Knochenersatzwerkstoffen ist jedoch, dass die Osteoblastenproliferation in unmittelbarer Nähe des alloplastischen Ersatzmaterialien zumeist reduziert ist. Osteoblasten und Osteoklasten sind aber notwendig für Knochenumbau, Knochenmatrixbildung, Knochenreifung und schließlich Heilung des Knochendefekts. Osteoklasten sind notwendig, für eine Resorption beziehungsweise den Abbau der alloplastischen Knochenersatzmaterialien. Die Osteoblasten für die Neubildung von Knochensubstanz entwickeln sich aus undifferenzierten Mesenchymzellen. Mesenchymzellen oder mesenchymale stromale Zellen sind sternförmig verzweigt und sie stehen über stehen über Zytoplasmafortsätze miteinander in mechanischer und kommunikativer Verbindung. Mesenchymalzellen differenzieren sich unter anderem in Osteoblasten. Sie lagern sich hautschichtartig an und sezernieren Kollagen, Calciumphosphate und Calciumcarbonate in den interstitiellen Raum, so dass neue Knochensubstanz entsteht. Osteoblasten kontrollieren auch die Funktion der Osteoklasten über den aktivierenden RANK-Ligand (RANKL) und das inhibierende Osteoprotogerin (OG), welches RANKL neutralisiert.

Ein Schritt zum Knochenzellwachstum liegt somit in der Differenzierung von mesenchymalen Zellen und Stammzellen zu Osteoblasten, welche die für Knochenmatrixbildung und Implantatintegration erforderlichen Proteine sezernieren. Die Neubildung von Knochenmatrix kann unter anderem durch das Auftreten nichtkollagener Knochenmatrixproteine wie BSP oder OPN (Osteopontin) nachgewiesen werden. Das Bone-Sialoprotein (BSP II) an sich ist ein Knochen-Glykoprotein wie Osteonectin und Osteopontin. Es stellt etwa 10% der nichtkollagenen Matrixproteine des Knochens, und es gibt viele Hinweise dafür, dass es eine Rolle im Knochenstoffwechsel besitzt und an der Differenzierung der mesenchymalen Stammzellen zu Osteoblasten beteiligt ist. Die Osteoblasten kontrollieren bekanntlich Knochenneubildung und Knochenresorption sowie die Knochenmineralisierung.

Die Primärstruktur von humanen BSP enthält eine Arg-Gly-Asp (RGD)-Sequenz für eine Integrin-vermittelte Zelladhäsion, so wie viele andere Proteine der extrazellulären Matrix und auch Fibronektin und Vitronektin. BSP kann auch ein Nukleus sein für eine Hydroxylapatit-Kristallisierung. BSP wird ansonsten von Zellen der osteoblastären Reihe gebildet und ist in heterotop mineralisierenden Bindegeweben vorhanden. Knochentumore, die zu Verkalkungen führen und Krebszellen, welche in Knochengewebe metastasieren, exprimieren regelmäßig BSP. So besteht zwischen der BSP-Expression von Brust- sowie Prostatakrebszellen und Knochenmetastasen eine stringente Kontrolle (siehe http://www.immundiagnostik.com/home/produkte/testkitsassays und dort genannte Referenzen).

Bei den hydraulischen Knochen-und Calciumphosphat-Zementen sind die mit metastabilen Calciumphosphaten bevorzugt, da diese in eine thermodynamisch stabilere Form übergehen. Zementsysteme mit kristalliner Phosphorsäure oder Calciumoxid hingegen können in der Umgebung extreme pH-Werte erzeugen. Bevorzugt sind CP-Zemente, die als Hauptbestandteile MCPM, MCPA, DCPA, DCPD, OCP, β-TCP, β-TCP, ACP, HA, CDHA, substituiertes HA, nicht stöchiometrisches HA, nanoHA, TTCP, CaSO₄, CaSO₄x 0,5 H₂O, CaSO₄x2H₂O, CaO, Ca(OH)₂ oder CaCO₃ oder deren Gemische enthalten. Für den medizinischen Einsatz sind auch Zemente beschrieben, die als Struvit aushärten; siehe EP 1296909 B1. Derartige Magnesium-AmmoniumPhosphat-Zemente bzw. Struvit-Zemente haben gute Kohäsion und einen steilen Festigkeitsanstieg nach dem Anmischen.

Auch die Füllstoffe sind wichtig, auch wenn sie nicht unmittelbar an der Zementreaktion beteiligt sind. Hydrogenphosphate haben eine hohe Löslichkeit und sind gute Puffer, für den Fall, dass die Abbindereaktion zu extremen pH-Werten führen könnte. In einer bevorzugten Ausführungsform enthält der partiell abgebundene Knochenzement als mineralische Feststoffe Calcium- oder Magnesiumhydrogenphosphat (CaHPO₄ oder MgHPO₄). Ein weiterer wichtiger Füllstoff ist Calciumcarbonat. Calciumsulfate hingegen sind Bindemittel für die Calciumphosphate, die zumeist unreaktiv sind.

Bei den Calciumphosphat-Zementen gibt es einige, deren Reaktionsprodukte Knochenmineralien ähnlich sind. Die sogenannten Brown/Chow-Zemente härten als Hydroxyapatit (HA) aus oder als Ca-defizienter HA (CDHA), substituierter HA, Nano-HA oder wenig kristalliner HA; siehe Brown WE, Chow LC in A new calcium phosphate water setting cement. In: Brown PW, editor. Cements research progress. Westerville, OH: American Ceramic Society; 1986. p. 352-79; US 5 336 264 A; US 6,117,456; US 5 262 166; EP 1 302 453 A1; Bohner M et al in Effect of several additives and their admixtures on the physico-chemica properties of a calcium phosphate cement. J Mater Sci Mater Med 2000, 11:111-116; Driessens FCM et al, in Effective formulations for the preparation of calcium phosphate bone cements, J Mater Sci Mater Med. 1994; 5: 164-170. Derartige Zemente haben eine sehr hohe Biokompatibilität, sind resorbierbar und nicht mutagen. Das Reaktionsprodukt besteht in der frühen Aushärtung aus Hydroxylapatit und bei Calciummangel aus Apatit (Ca₉[HPO₄][PO₄]₅[OH]). Phosphationen in der Anmischflüssigkeit erhöhen die Hydroxylapatit-Bildung. Hydroxylapatit (HA) ist unter physiologischen Bedingungen stabil und wird nur zellulär abgebaut. Leichter löslich ist Dicalciumphosphatanhydriddihydrat (DCPD). Calcium-phosphat-Zemente mit DCPD sind für Anwendungen bevorzugt, wo eine schnelle Auflösung des Materials gewünscht ist. Übliche Ausgangsmaterialien für DCPD-Zemente sind α-TCP und Monocalciumphosphat.

Für die Abbindereaktion werden die gemahlenen Feststoffpartikel dispergiert und in engen Kontakt gebracht, wobei sie sich in der gewählten Flüssigkeit partiell lösen, umkristallisieren und umpräzipitieren. Ergänzend erfolgt ein Vermischen zu einer spritzbaren oder druckbaren Paste im Sinne eines physikalischen Gemisches, wobei eine große Kontaktoberfläche zwischen den Phasen hergestellt wird. Die Ausbildung und Stabilität des physikalischen Gemisches hängt dabei nicht nur von den Komponenten ab, sondern auch von der beim Vermischung eingebrachten Energie und den Einsatz oberflächenaktiver Substanzen.

Die Verwendungsmöglichkeiten der Knochenzementkörper sind vielfältig und reichen von Knochenteilen zur Fixierung von Implantaten, einer Verstärkung von mechanisch hoch belasteten Knochen bis zur Rekonstruktion von Skelettstrukturen. Der große Vorteil der im 3-D-Drucker hergestellten Knochenzementkörper mit interkonnektierenden Poren ist die Anpassung von Form und Eigenschaften an die jeweilige Verwendung. Neben der Art der Feststoffe spielen hier vor allem die Partikelgröße und -form eine entscheidende Rolle. Die Pasten zur Herstellung der Knochenzementkörper enthalten die mineralischen Feststoffe zu 25% bis 75% (Gewichtsprozent). Die Feststoffpartikel liegen in hochdisperser Form vor, wie dies beispielsweise für nanodimensionierte Calciumphosphate, Calciumcarbonate, Calciumhydroxide, Calciumoxide, Calciumsulfate, Magnesiumoxide, Magnesiumhydroxyd-Carbonate, Silikate verschiedener Kationen und durch Sol/Gel-Verfahren hergestellte reaktive Gläser gegeben sein kann. Die hohe Porosität des Knochenzementkörpers erleichtert Knochenneubildung, das Einwachsen von Osteoblasten und -klasten, Knochenumbau und Neubildung. Letzteres wird unterstützt durch adsorbiertes BSP auf den Strängen und in den Poren des partiell gehärteten Knochenzementkörpers. Da das Knochenersatzmaterial mindestens teilweise abgebunden ist, kommt es somit zu keiner Freisetzung von Calciumionen mehr, welche die Aktivität des aufgebrachten BSP mindern könnte und auch zu keiner vorzeitigen Kristallisation von Hydroxylapatit.

### BEISPIELE

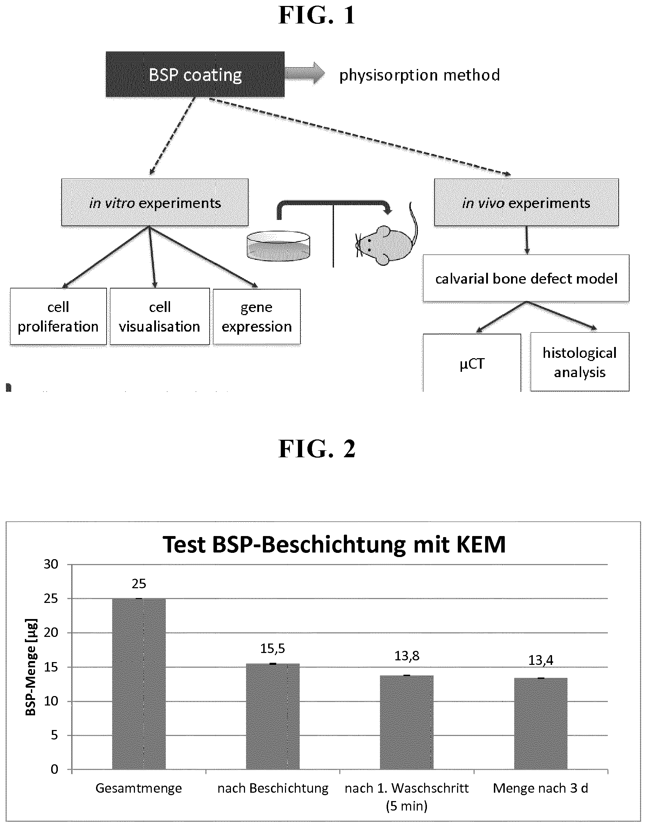

Fig 1 zeigt den prinzipiellen Aufbau der Versuche mit den allotropen Knochenersatzmaterialien und Knochenzementkörpern.

### Beispiel 1 Austesten der Knochenersatzmaterialien und BSP-Physisorption

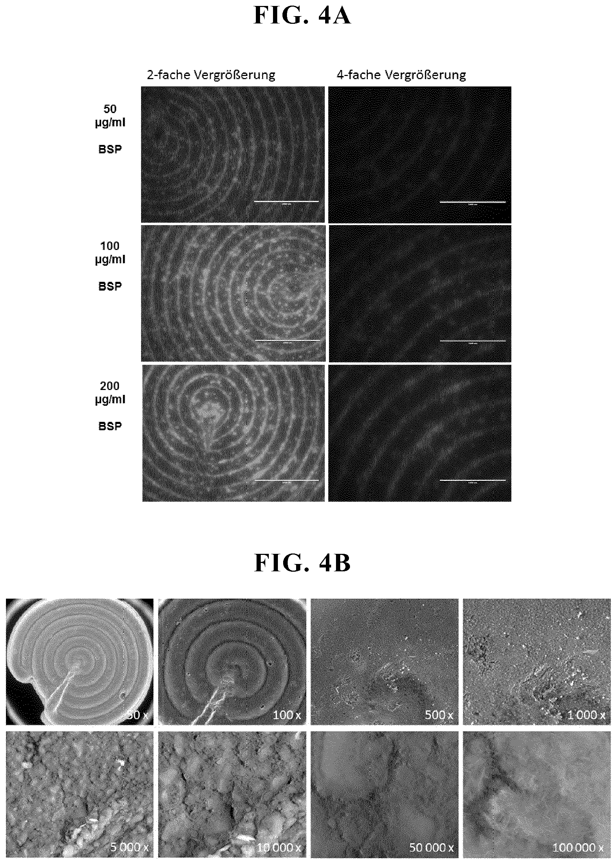

Es wurde ein Knochenzement gemäß WO 2015/059240 (Beispiel 1) eingesetzt. Das Calciumphosphatzement-Pulver bestand aus einem Gemisch von vermahlenem α-Tricalciumphosphat, Calciumhydrogenphosphat, Calciumcarbonat, und Hydroxylapatit. Die Paste für den 3-D-Druck enthielt zudem als Puffer Kaliummonohydrogenphosphat sowie eine Öl-Emulgator-Mischung von kurzkettigen Triglyceriden, Castorölethoxylat und Cetylphosphat. Das Gemisch wurde zu einer plastisch verformbaren Paste vermengt. Diese wurde dann in einem handelsüblichen 3-D-Drucker strangweise zu formfesten Knochenzementkörpern gedruckt. Die Porosität des Formkörper ergibt sich aus den gewählten Strangabständen (siehe **Figuren 4B****,** **4C**)

Nach dem Drucken wurde der Knochenzementkörper für ein hydraulisches Abbinden in eine künstlich feuchte Umgebung gebracht. Der Abbindevorgang wurde abgebrochen durch Waschen des Körpers mit wasserfreiem Aceton. Der Formkörper wurde nach bei 80 Grad Celsium im Trockenschrank getrocknet, durch Gammabestrahlung sterilisiert und trocken unter Schutzgas bis zur Anwendung aufbewahrt (siehe Figuren 4B-D).

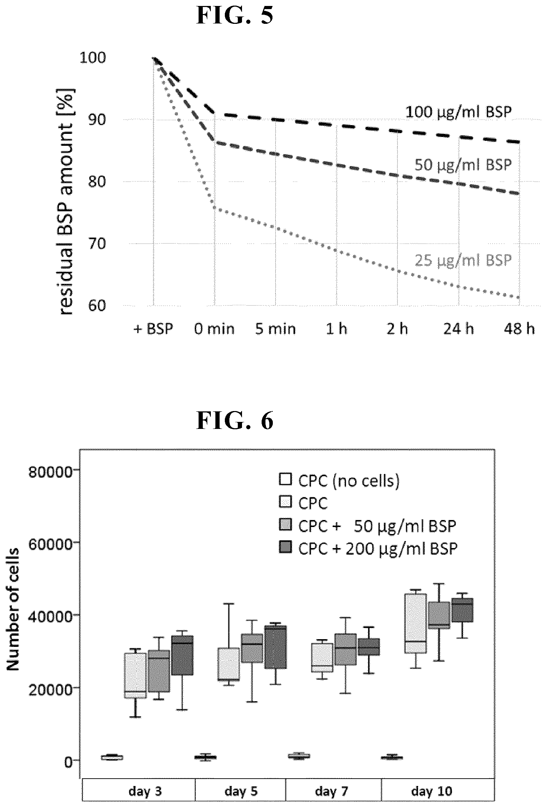

Wurde das so hergestellte Knochenersatzmaterial in Medium gebracht, war keine pH-Änderung festzustellen bzw. der pH blieb im physiologischen Bereich. Dies bedeutet, dass keine Freisetzung von Calciumionen mehr erfolgte. Dann ermittelten wir ein bestes Verfahren zur Physisorption von bioaktivem BSP des CPC-Körpers (KEM). Physisorption auf einer Oberfläche sollte die Bioaktivität und Bioverfügbarkeit des BSP am wenigsten beeinflussen. Vorversuche mit fluoreszenzmarkiertem BSP (BSP mit Fluorescein gekoppelt) und Freisetzungsversuche zeigten (siehe graphisch dargestellte Ergebnisse in **Figuren 2****,** **3****,** **4a****,** **5**), dass durch Physisorption eine lokale Menge an BSP auf dem KEM erreicht werden kann. Hierzu wurden auch die beste BSP-Konzentration in der Lösung und die Inkubationszeit ermittelt (siehe **Fig. 5**). Für die Physisorption des BSP auf den CPC-Materialien wurden unterschiedliche BSP-Konzentrationen (25 µg/ml, 50 µg/ml und 100 µg/ml) getestet und die BSP-Menge auf dem KEM nach mehreren Waschschritten. Die Ergebnisse sind in den **Fig. 2****,** **4a** **und** **5** zusammengefasst.

Siehe **Fig. 2** und **Fig. 5****.** Höhere BSP-Konzentrationen lieferten ein besseres Ergebnis (mehr verbleibendes BSP auf dem KEM); siehe auch **Fig. 4a**. Die Physisorption von BSP erfolgte schließlich nach Waschen mit Albumin-haltiger Lösung und Tränken der Körpers über Nacht (ca. 18 h) bei Raumtemperatur mit BSP-haltiger Lösung (50 µg BSP in N-Methylpyrrolidon/PBS).

Für die Untersuchung der BSP-Freisetzung vom KEM nach Physisorption war das BSP mit Fluorescein markiert. Abnahme der Beschichtungslösung (Probe 0); Zugabe von 500 µl PBS, Inkubation für 5 min, anschließend Abnahme der Waschlösung (Probe 1 = erster Waschschritt); Zugabe von 1 ml PBS, Inkubation für 3 d (Probe 2 = nach 3 d). Die Ergebnisse sind in nachstehender Tabelle 1 und in **Fig. 2** zusammengefasst.

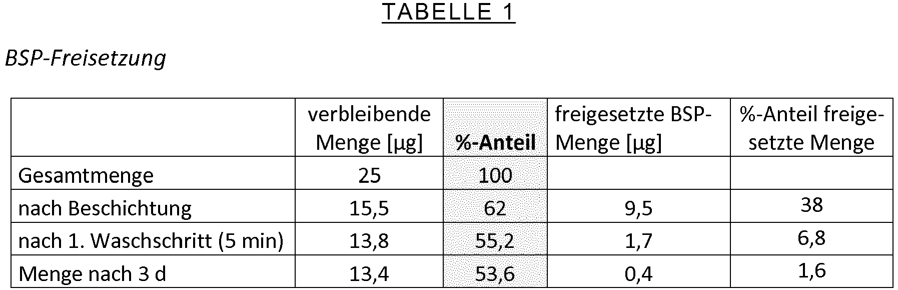

### Beispiel 2 Physiologische Versuche

Die Versuche erfolgten mit rekombinantem humanen BSP, exprimiert und isoliert aus einer stabilen CHO-Zellline (Immundiagnostik AG, Bensheim, DE). Die für die Zellkulturversuche verwendeten primären humanen Osteoblasten (hOBs) wurden aus menschlichen Proben isoliert. Sie fielen ab aus Operationen bei einem Ersatz der Hüfte oder des Knies. Alle Patienten erteilten schriftliches Einverständnis zur Verwendung des gewonnenen Materials und die Versuche waren vom zuständigen Ethickommitee genehmigt.

Die Zellen wurden wie anderweitig beschrieben isoliert; siehe Hofmann A et al in Biomaterials 2008, 29 (31), 4217-4226 und Bone 2008, 42 (5), 894-906. Die Knochenfragmente aus der Schraube wurde in PBS gewaschen und 45 Minuten bei 37°C mit Typ 1-Collagenase verdaut. Nach weiterem Waschen mit PBS wurden sie kultiviert in DMEM/F12 (Gibco®, Life Technologies, Grand Island, NY, US) mit 20 % fötalem Kälberserum (Biochrom AG, Berlin, DE), 1 % Penicillin und Streptomycin. Nach der ersten Passage wurden die humanen Osteoblasten in diesem Medium auch weiterkultiviert, mit Mediumwechsel alle 14 Tage. Das Medium für die Differenzieruung enthielt 10⁻⁸ M Dexamethason, 3,5 mM β-Glycerolphosphat und 50 µg/ml Ascorbinsäure. L929 Fibroblasten als Helferzellen wurden kultiviert in MEM Earl's medium, supplementiert mit 10% fötalem Kälberserum, 1% Penicillin und Streptomycin. Die Zellkultur erfolgt unter feuchter Atmosphäre mit 5 % CO₂ at 37°C. Zur Bestimmung der Transduktionseffizienz und für Versuche mit den Zellen unter dem Mikroskop wurden die humanen Osteoblasten einmal transduziert mit dem Lentivirus-Vektor SEW-eGFP für gemäß Standardprotokoll; siehe Brendel C et al, Gene Ther. 2012, 19(10):1018-1029; Kuhn S et al, J. Mater. Sci. Mater. Med. 2014, 25:2549-2560. Die Transduktionseffizienz wurde durch Flusszytometrie bestimmt.

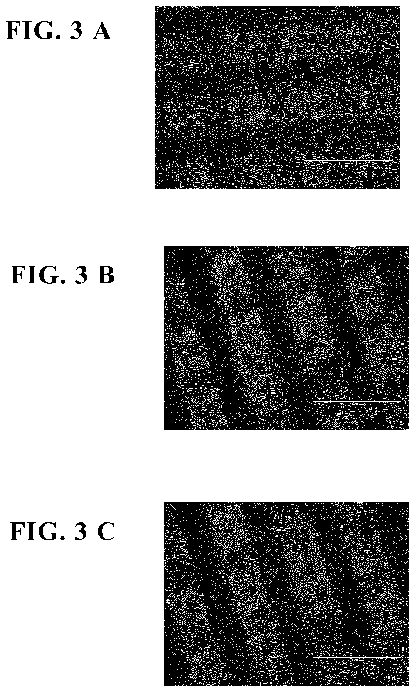

*Zellaussaat auf KEM:* Die Aussaat von humanen Osteoblasten (Markierung mit Cell Tracker Rot) in 200 µl Medium; nach 2 h Zugabe von weiteren 300 µl Medium; visuelle Kontrolle am EVOS (siehe Fig. 3A-C). Humane Osteoblasten zeigten auf BSP-beschichteten Knochenersatzmaterialien (CPC + 200 µg/ml BSP) tendenziell eine höhere Proliferationsrate im Vergleich zum Zellwachstum auf unbeschichteten KEM.

**Figur 6** zeigt im Ergebnis die Proliferation der hOB auf CPC ± BSP an den Tagen 3, 5, 7 und 10 nach Zellaussaat. Für die Beschichtung der Knochenersatzmaterialien wurden unterschiedliche BSP-Konzentrationen (50 µg/ml und 200 µg/ml) getestet. Als Negativkontrolle dienten unbehandelte Knochenersatzmaterialien. Die Unterschiede waren signifikant an Tag 3 [KEM - KEM + 200 µg/ml ^{∗∗∗}(p = 0.000) und bei KEM + 50 µg/ml - KEM + 200 µg/ml ^{∗} (p = 0,020)] und 5 [KEM - KEM + 200 µg/ml ^{∗} (p = 0.048)]. Die Negativkontrolle (ohne humane Osteoblasten) zeigte an allen Tagen einen signifikanten Unterschied im Vergleich zu den anderen Gruppen (*** p = 0,000). Die Statistik erfolgte mit einem ONE-WAY-ANOVA Test und anschließendem Vergleich mittels Mann-Whitney-U-Test.

### Beispiel 3 SEM - Rasterelektronenmikroskop-Analyse

Visualisierung der Knochenersatzmaterialien mit Hilfe des Rasterelekronenmikroskops. Darstellung der CPC-Scheiben (in vitro Versuche) - linke Seite - und der CPC-Zylinder (für die in vivo Versuche) - rechte Seite - bei zwei Vergrößerungen (10.000x und 50.000x), Verwendete Knochenersatzmaterialien (KEM) Calcium-Phosphat-Cement (CPC), CPC-Scheiben (in vitro) und CPC-Zylinder mit interkonnektierenden Poren (in vivo Versuche). Die Struktur der CPC-Scheiben und der CPC-Zylinder wurde mittels Rasterelektronenmikroskop analysiert und verglichen. Auf mikroskopischer Ebene gleicht sich die Struktur der verwendeten KEM.

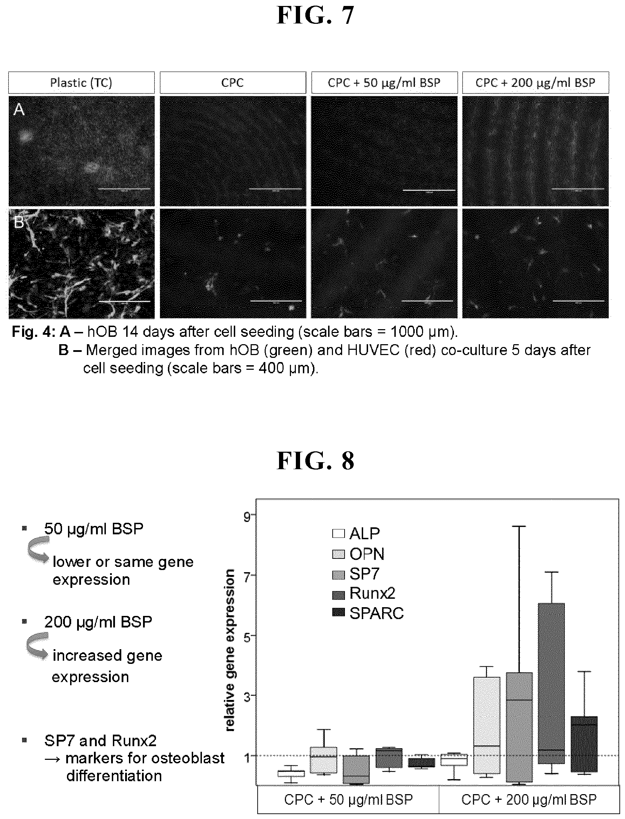

Siehe **Figur 7**. Humane Osteoblasten wurden auf unbeschichteten und auf mit BSP-beschichteten Knochenersatzmaterialien ausgesät. Nach 14 Tagen wurden die Osteoblasten mit Hilfe von Calcein AM grün markiert. Bei der Zelladhäsion von humanen Osteoblasten war kein Unterschied zwischen unbeschichteten und beschichteten KEM quantitativ erkennbar. Qualitativ war eine verstärkte Zelladhäsion auf der Positivkontrolle (tissue-culture treated plastic dishes) sichtbar.

Visualisierung von humanen Osteoblasten auf CDHA-Scheiben mittels EVOS Fluoreszenzmikroskop. CDHA-Scheiben (KEM), Beschichtung mit BSP in ansteigender Konzentration, Färbung mit Calcein AM nach 14 Tagen hOB-Proliferation (Scale bar = 1000 µm).

### Beispiel 4 Genexpressionsanalyse mittels gRT-PCR

*Einfluss auf die Genexpression.* Das KEM mit einer Beschichtung bei hoher BSP-Konzentration (200 µg/ml) hatte einen Einfluss auf die Genexpression von hOB im Vergleich zum unbeschichteten KEM; siehe **Figur 8****.** Die Beschichtung mit niedrigerer BSP-Konzentration (50 µg/ml) hatte keinen Einfluss bzw. teilweise eine Verringerung der Genexpression in den hOBs im Vergleich zur Kontrolle.

Genexpressionsanalyse 14 Tage nach Aussaat der hOB auf unbeschichtete und BSP-beschichtete KEM. Für die Beschichtung der KEM wurden unterschiedliche BSP-Konzentrationen (50 µg/ml und 200 µg/ml) geprüft. Die hOB Genexpression der Kontrolle (CPC) ist als gestrichelte Linie dargestellt.

Zwischen den einzelnen Gruppen (unbeschichtetes KEM, KEM + 50 µg/ml BSP, KEM + 200 µg/ml BSP) gibt es keine signifikanten Unterschiede (n.s.) bei den Markern OPN, SP7, Runx2 und SPARC. Einziger signifikanter Unterschied bei ALP: Die Beschichtung mit 50 µg/ml BSP zeigt eine signifikante Verringerung der ALP-Genexpression von humanen Osteoblasten im Vergleich zur Kontrolle (**, p = 0,008) und der Beschichtung mit hoher BSP-Konzentration (*, p = 0,015). Die Berechnung der Statistik erfolgte je Genemarker mit einem ONE-WAY-ANOVA Test und anschließendem Vergleich mittels Mann-Whitney-U-Test.

Unsere Versuche zeigten eine konzentrationsabhängige Steigerung der Proliferation und Genexpression bei humanen Osteoblasten auf BSP-beschichteten Knochenersatzmaterialien (KEM). Unsere in vitro-Versuche führten zu dem Ergebnis, bei in in vivo Versuchen die KEM und Knochenzementkörper mit einer konzentrierte BSP-Lösung (200 µg/ml) zu behandeln.

### Beispiel 5 Kalotten-Bohrlochdefekt-Modell (Maus)

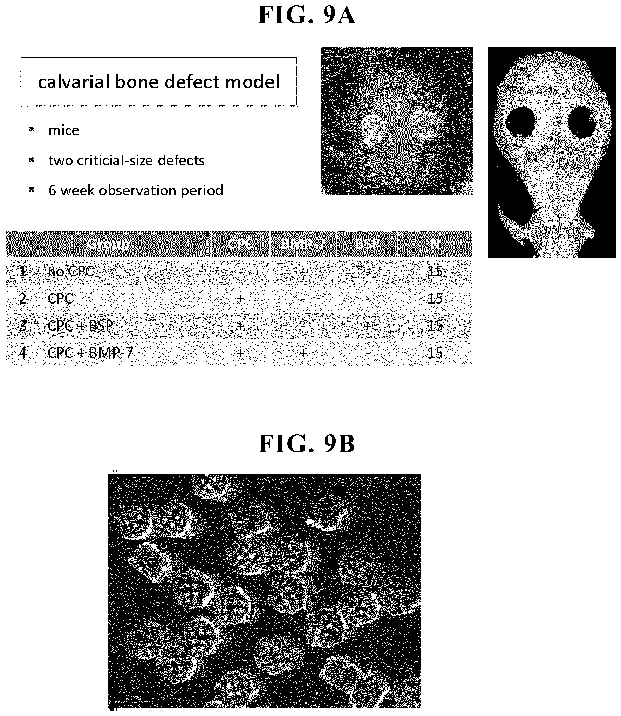

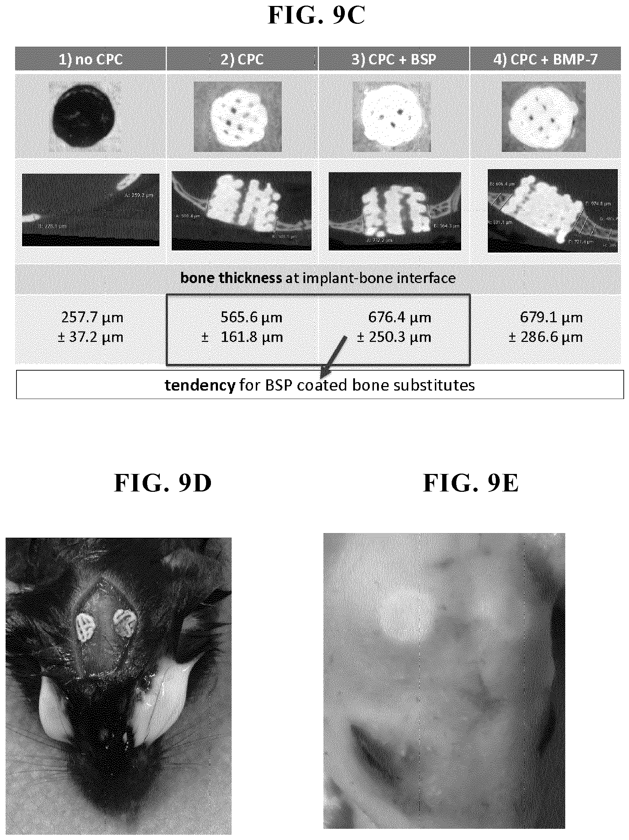

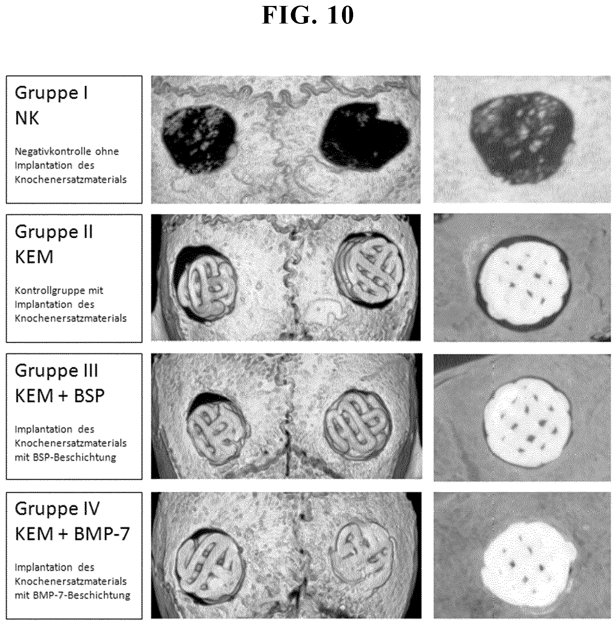

Siehe **Figuren 9A-E****,** und **Fig. 10****.** Der Tierversuch wurde vom zuständigen Landesuntersuchungsamt genehmigt. Für den nachstehenden Kalotten-Modell-Tierversuch wurden insgesamt 60 Tiere eingesetzt. Die Mäuse August 2015 wurden unter Vollnarkose operiert und in ihre Kalotte jeweils zwei Bohrlöcher mit 2,5 mm Durchmesser gesetzt - also ein Knochendefekt kritischer Größe. Dann wurde in das Bohrloch je nach Gruppe der CPC-Knochenzementkörper mit interkonnektierenden Poren eingesetzt. Der Durchmesser des Zylinders des Knochenzementkörpers betrug ca. 2,4 mm. **Figur 9B** zeigt eine Mikroskopaufnahme des in-vivo verwendeten zylindrischen Knochenzementkörpers. Diese waren vor der Operation durch Physisorption mit BSP bzw. BMP-7 wie oben in den Beispielen 1 bis 4 beschichtet. Nach einem Beobachtungszeitraum von 8 Wochen wurden die Tiere mit Kohlendioxid betäubt und enthauptet. Die Schädel wurden eine Woche in Formalin fixiert und für die µCT-Aufnahmen vorbereitet. Der Tierversuch umfasste vier Gruppen (**Fig. 9C**):
a) Tiere mit unbehandeltem Bohrlochdefekt (vernäht, ohne Implantation eines Knochenzementkörpers) für eine negative Kontrolle;
b) Tiere mit implantiertem Knochenzementkörper ohne BSP-Beschichtung;
c) Tiere mit implantiertem Knochenzementkörper mit BSP-Beschichtung;
d) Tiere mit implantiertem Knochenzementkörper, beschichtet mit BMP-7, als positive Kontrolle.

Tabelle 2 und **Fig. 9A** zeigt die genaue Gruppeneinteilung der Tiere.

**TABELLE 2**

| **Gruppe** | **KEK** | **BSP** | **BMP** | **N Tiere** | **Zeitpunkt** |
|---|---|---|---|---|---|
| 1 | - | - | - | 15 | 8 Wochen |
| 2 | + | - | - | 15 | 8 Wochen |
| 3 | + | + | - | 15 | 8 Wochen |
| 4 | + | - | + | 15 | 8 Wochen |

| | | | | | |
|---|---|---|---|---|---|
| (+) seht für einen Knochenzementkörper (KEK) mit Beschichtung mit BSP bzw. BMP (-) Verwendung ohne KEK, BSP oder BMP | | | | | |

Figur 9C und Fig. 10 zeigen µ-CT-Aufnahmen der Kalotten. Nach 8 Wochen post-operativen Beobachtungszeit wurden post-mortem die Bohrlochdefekt durch µCT untersucht. In die Auswertung für die Bestimmung von neuem Knochenvolumen bezogen auf das Defekt-Gesamtvolumen kamen die Tiere, bei denen ein Kontakt vorhanden war zwischen Knochenzementkörper und Knochen. Gemessenes Defektvolumen (total) = 35,826 mm³; Ø Zylinderhöhe: 2,25 mm; Ø Gemessene Durchmesser (total): 4,5 mm.

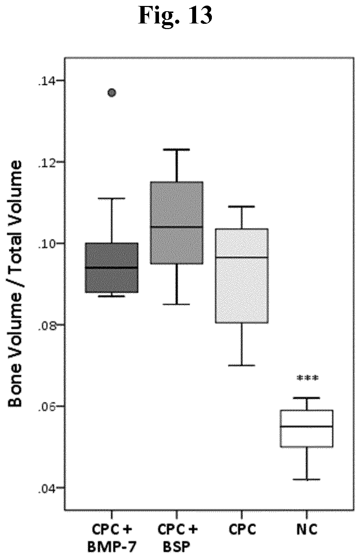

Die Ergebnisse sind in dem Diagramm von **Figur 13** darstellt. Wegen der geringen Zahl der Tiere waren die Unterschiede zwischen unbehandeltem CPC-Knochenzementkörper (CPC), Ersatzkörper mit adsorbiertem BSP (CPC + BSP) und Ersatzkörper mit adsorbiertem BMP-7 (CPC + BMP-7) als deutliche Tendenz feststellbar, trotz des nivellierenden langen Zeitraums. Die Wirkung des BSP auf das Knochenwachstums ist vor allem initial, in den ersten Wochen liegend. Ein signifikanter Unterschied besteht zwischen der Negativkontrolle (keine Behandlung, NC = negative control) und den Vergleichsgruppen (p < 0.001, ***).

Die µCT zeigte im Ergebnis tendenziell erhöhte Knochenbildung und einen Umbau der behandelten Knochenzementkörper im Bohrlochdefekt gegenüber unbehandelten Körpern. Die Beschichtung mit BSP oder BMP begünstigt die Defektheilung insgesamt. Die tendenziell stärkste Knochenneubildung, bestimmt über Knochenvolumen und Knochendichte, wurde bei einem CPC-Körper mit adsorbiertem BSP beobachtet (siehe **Fig. 9C**). Die Wirkung des BSP erwies sich in diesem Versuch als stärker als bei der Positivkontrolle mit BMP-7.

### Beispiel 6 Messung der Knochendicke an der Implantat-Grenzfläche

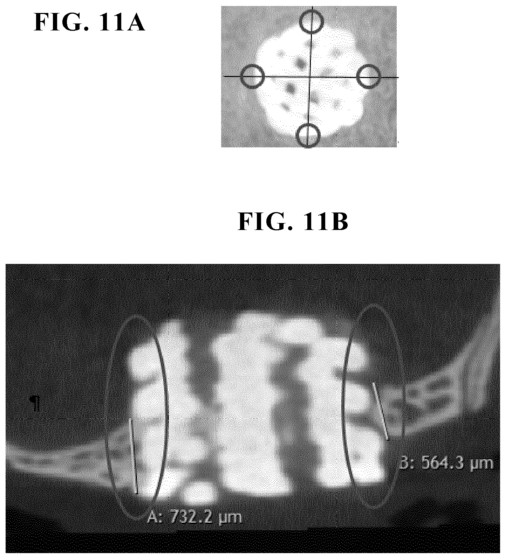

Alternativ zur Bone-Volume/Total-Volume Auswertung, wurden die Knochendicken an der Implantat-Knochen-Grenzfläche vermessen; siehe Figs. 11A+B. In die Auswertung kamen die Defekte, bei denen eine Kontaktfläche zwischen Knochenersatzmaterial (KEM) und Knochen bestand (NK n = 8, KEM n = 8, KEM + BMP-7 n = 9, KEM + BSP n = 10).

**TABELLE 3**

| *Defekt mit Kontaktfläche zwischen KEM* + *Knochen* | | | |
|---|---|---|---|
| 1) Negativkontrolle | 2) KEM | 3) KEM + BSP | 4) KEM + BMP-7 |
| 257,7 µm | 565,6 µm | 676,4 µm | 679,1 µm |
| ± 37,2 µm | ± 161,8 µm | ± 250,3 µm | ± 286,6 µm |

| | | | |
|---|---|---|---|
| Messung der Knochendicken an den Achsen 4 Messpunkte für die Knochendicke (grüne Kreise) pro Defekt | | | |

Tendenziell führen die offenbarten CPC-Knochenzementkörper mit adsorbiertem BSP zu verstärkter Knochenneubildung als unbehandelte Ersatzkörper. Für eine Bestimmung der statistischen Signifikanz war die Tierzahl zu gering. Ähnlich der Vergleich mit der BMP-7 behandelten Positivkontrolle (KEM + BMP-7). Bei allen Knochenzementkörper (KEM, KEM + BSP, KEM + BMP-7) war eine signifikant größere Knochenneubildung feststellbar im Vergleich zur Negativkontrolle.

### Beispiel 7 Histologie

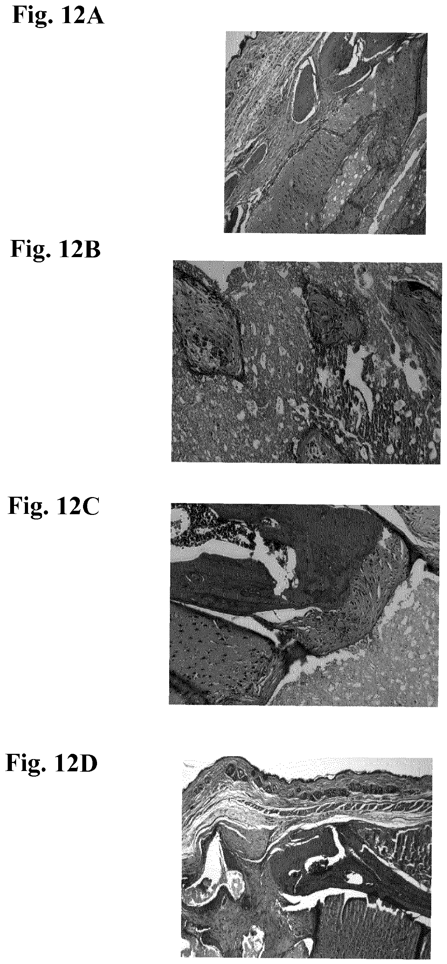

Die Knochenzementkörper wurden nach dem Tierversuch verschieden histologisch untersucht, durch a) Paraffineinbettung ohne Entkalkung; b) Paraffineinbettung nach 3 Wochen Entkalkung, c) Einbettung in Technovit® (Cutting-Grinding Technique). Methode a) führte zu unsauberen Schnitten und schlechten histologischen Darstellungen. Abb. 12A-D zeigt Darstellungen nach einer Hämatoxylin-Eosin-Färbung gemäß Methode b. Die Schnitte bzw. Strukturen sind gut dargestellt. Da die Entkalkung der Untersuchungsprobe mittels EDTA-Lösung (Entzug von Calcium) und ihr Einfluss auf die Struktur des Knochenersatzmaterials problematisch war, wurden die Proben für die weitere Untersuchung noch in Technovit® eingebettet, was insgesamt bessere Ergebnisse lieferte.

### Diskussion und Zusammenfassung der Ergebnisse

Es ist überraschend, dass selbst nach der langen Beobachtungszeit von 8 Wochen ein Einfluss des physisorbierten BSP im hydraulisch abgebundenen Konchenersatzmaterial beobachtet war und dass es tendenziell verstärkten Knochenumbau und Knochenheilung bewirkte. Dies bedeutet, dass auf hydraulisch abgebundenem Knochenzement das BSP nicht denaturiert und inaktiviert wird wie beispielsweise auf Calcium-abgebenden Hydroxyapatit. Auch ist in der chirurgischen Verwendung als Knochenersatzmaterial eine Deaktivierung des BSP durch das Komplementsystem nicht zu befürchten, so dass eine verbesserte Verwendung in der Chirurgie vorliegt.

## Patentansprüche

1. Strukturfester Knochenersatzkörper aus mineralischem Calciumphosphatzement mit verbleibender hydraulischer Aktivität, enthaltend einen Anteil an abgebundenem mineralischen Calciumphosphatzement und einen Anteil an nicht-abgebundenem hydraulisch reaktiven Calciumphosphatzement, **dadurch gekennzeichnet, dass** der Knochenersatzkörper aus Zementsträngen mit interkonnektierenden Poren aufgebaut ist, dass nach dem Aufbau ein Schritt mit hydraulischem Abbinden und Härten der Stränge folgt, und dass auf der hydraulisch abgebundenen, gehärteten Oberfläche und in den Poren des Ersatzkörpers, der im Wesentlichen nunmehr gegenüber Proteinen inert ist, durch Physissorption humanes Bone-Sialoprotein aufgebracht ist.

2. Knochenersatzkörper nach Anspruch 1, wobei vor Physisorption des humanen Bone-Sialoprotein neutrale globuläre Proteine adsorbiert werden, die nicht aus dem Komplementsystem stammen.

3. Knochenersatzkörper nach Anspruch 1 oder 2, wobei vor Physisorption mit humanen Bone-Sialoprotein die Zementstränge mit synthetischem Poly(Glu) vorbehandelt werden, allein oder zusammen mit Poly(Lys), Poly(Gly-Pro-Hyp), Tropocollagen oder Gelatine.

4. Knochenersatzkörper nach einem der Ansprüche 1 bis 3, **dadurch gekennzeichnet, dass** der Knochenersatzkörper eine Gesamtporosität von 20 bis 90% besitzt.

5. Knochenersatzkörper nach einem der Ansprüche 1 bis 4, wobei der Knochenzement β-Tricalciumphosphat-Granula enthält, wobei die einzelnen Granula eine Größe von >100 Mikrometer aufweisen.

6. Knochenersatzkörper nach einem der Ansprüche 1 bis 5, enthaltend ein Knochenzementmaterial, das Feststoffe enthält und mindestens 10% der Feststoffe eine Partikelgröße < 10 µm und mindestens 10% der Feststoffe eine Partikelgröße > 50 µm aufweisen.

7. Knochenersatzkörper nach einem der Ansprüche 1 bis 6, **dadurch gekennzeichnet, dass** er die strukturfeste Gestalt eines gedruckten 3D-Formkörpers aufweist.

8. Stränge oder formbare Paste mit einem Anteil an abgebundenem mineralischen Calciumphosphatzement und einem Anteil an nicht-abgebundenem hydraulisch reaktiven Calciumphosphatzement gemäß einem der Ansprüche 1 bis 6, auf die bioaktives BSP sorbiert ist, alleine oder zusammen mit natürlichen oder synthetischen Proteinen gemäß einem der vorstehenden Ansprüche 2 bis 7.

9. Strukturfester Knochenersatzkörper gemäß einem der Ansprüche 1 bis 7 zur Anwendung in der rekonstitutiven Chirurgie.

10. Verfahren zur Herstellung eines strukturfesten Knochenersatzkörpers gemäß einem der Ansprüche 1 bis 7, **dadurch gekennzeichnet, dass** Stränge einer Paste gemäß Anspruch 8 mit einem 3-Drucker in Gestalt eines porösen strukturfesten Körpers aufgebaut werden.

## Claims

1. A structure-stable bone graft of calcium phosphate mineral cement with residual hydraulic activity containing a proportion of set calcium phosphate mineral cement and a proportion of non-set hydraulically reactive calcium phosphate cement, **characterized in that** the bone graft is constructed of cement strands and with interconnected pores, **in that** a step of hydraulic setting and hardening of the strands follows after construction, and **in that** human bone sialoprotein is applied by physisorption onto the hydraulically hardened surface and in the pores of the graft, which is now essentially inert towards proteins..

2. Bone graft according to claim 1, wherein neutral globular proteins not from the complement system are adsorbed prior to the physisorption of the human bone sialoprotein.

3. Bone graft according to claim 1 or 2, wherein the cement strands are pre-treated with synthetic poly(Glu), alone or together with poly(Lys), poly(Gly-Pro-Hyp), tropocollagen or gelatine prior to the physisorption of the human bone sialoprotein.

4. Bone graft according to any claim 1 to 3, **characterized in that** the bone graft has a total porosity of 20 to 90%.

5. Bone graft according to any claim 1 to 4, wherein the bone cement contains granules of β-tricalcium phosphate wherein the individual granules have a size of greater than 100 micrometers.

6. Bone graft according to any claim 1 to 5 and containing a bone cement material which contains solids wherein at least 10% of the solids have a particle size of less than 10 micrometers and at least 10% of the solids have a particle size of greater than 50 micrometers.

7. Bone graft according to any claim 1 to 6 **characterized in that** it is a structure-stable 3D-printed shaped body.

8. Strands or moldable paste comprising a proportion of set calcium phosphate mineral cement and a proportion of unset hydraulically reactive calcium phosphate cement according to any claim 1 to 6, onto which bioactive BSP can be sorbed, alone or together with natural or synthetic proteins according any claim 2 to 7.

9. Structure-stable bone graft according to any claim 1 to 7 for use in reconstitutive surgery.

10. Process for preparing a structure stable bone graft according to any claim 1 to 7, **characterized in that** strands of a paste according to claim 8 are made up by a 3D-printer in the form of a porous structure-stable body.

## Revendications

1. Corps de substitution osseuse à structure rigide en ciment minéral à base de phosphate de calcium avec une activité hydraulique résiduelle, contenant une proportion de ciment minéral à base de phosphate de calcium ayant pris et une proportion de ciment à base de phosphate de calcium à réactivité hydraulique n'ayant pas pris, **caractérisé en ce que** le corps de substitution osseuse est constitué de brins de ciment avec des pores interconnectés, **en ce que** l'assemblage est suivi d'une étape de prise hydraulique et de durcissement des brins, et **en ce qu'**une sialoprotéine osseuse humaine est appliquée par adsorption physique sur la surface durcie ayant pris de manière hydraulique et dans les pores du corps de substitution qui devient alors essentiellement inerte par rapport aux protéines.

2. Corps de substitution osseuse selon la revendication 1, dans lequel des protéines globulaires neutres qui ne sont pas issues du système du complément sont adsorbées avant l'adsorption physique de la sialoprotéine osseuse humaine.

3. Corps de substitution osseuse selon la revendication 1 ou 2, dans lequel les brins de ciment sont prétraités avec de la poly(Glu) synthétique, seul ou conjointement avec de la poly(Lys), de la poly(Gly-Pro-Hyp), du tropocollagène ou de la gélatine, avant l'adsorption physique avec de la sialoprotéine osseuse humaine.

4. Corps de substitution osseuse selon l'une quelconque des revendications 1 à 3, **caractérisé en ce que** le corps de substitution osseuse a une porosité totale comprise entre 20 et 90 %.

5. Corps de substitution osseuse selon l'une quelconque des revendications 1 à 4, dans lequel le ciment osseux contient des granules de phosphate de β-tricalcium, dans lequel les granules individuels présentent une taille > 100 µm.

6. Corps de substitution osseuse selon l'une quelconque des revendications 1 à 5, contenant un matériau de ciment osseux, **caractérisé en ce qu'**il contient les substances solides et au moins 10 % des substances solides présentent une taille de particules < 10 µm et au moins 10 % des substances solides présentent une taille de particules > 50 µm.

7. Corps de substitution osseuse selon l'une quelconque des revendications 1 à 6, **caractérisé en ce qu'**il présente la configuration à structure rigide d'un corps moulé en 3D imprimé.

8. Brins ou pâte pouvant être moulée, avec une proportion de ciment minéral à base de phosphate de calcium ayant pris et une proportion de ciment à base de phosphate de calcium à réactivité hydraulique n'ayant pas pris selon l'une quelconque des revendications 1 à 6, sur lesquels ou laquelle est adsorbée la BSP (sialoprotéine osseuse) bioactive, seul ou conjointement avec des protéines naturelles ou synthétiques selon l'une quelconque des revendications précédentes 2 à 7.

9. Corps de substitution osseuse à structure rigide selon l'une quelconque des revendications 1 à 7, destiné à une utilisation en chirurgie reconstructive.

10. Procédé de fabrication d'un corps de substitution osseuse à structure rigide selon l'une quelconque des revendications 1 à 7, **caractérisé en ce que** des brins d'une pâte selon la revendication 8 sont assemblés avec une imprimante 3D dans la configuration d'un corps poreux à structure rigide.
